(19) Europäisches Patentamt
European Patent Office
Office européen des brevets

(11) Publication number: 0 256 978 B1

(12) **EUROPEAN PATENT SPECIFICATION**

(45) Date of publication of patent specification: 23.01.91

(51) Int. Cl.$^5$: C 07 D 249/18, C 23 F 11/14, C 10 M 133/44

(21) Application number: 87810426.4

(22) Date of filing: 27.07.87

# (54) A method of inhibiting corrosion on metal surfaces using perfluoroalkyl containing triazoles.

(30) Priority: 31.07.86 US 892217
31.07.86 US 892219

(43) Date of publication of application:
24.02.88 Bulletin 88/08

(45) Publication of the grant of the patent:
23.01.91 Bulletin 91/04

(84) Designated Contracting States:
BE DE FR GB IT NL

(56) References cited:
EP-A-0 088 724
FR-A-2 026 493
US-A-4 132 660
CHEMICAL ABSTRACTS, vol. 90, no. 24, 11th June 1979, page 593, abstract no. 195580c, Columbus, Ohio, US & JP A 87141622
CHEMICAL ABSTRACTS, vol. 82, no. 11, 17th March 1975, page 433, abstract no. 72882e, Columbus, Ohio, US; A. KREUTZBERGER et al.: "Synthesis and antiinflammatory activity of aliphatically substituted benzotriazoles"

(73) Proprietor: CIBA-GEIGY AG
Klybeckstrasse 141
CH-4002 Basel (CH)

(72) Inventor: Clark, Kirtland P.
10 Allan Way
Bethel Connecticut 06801 (US)
Inventor: Karydas, Athanasios
574 92nd Street
Brooklyn New York 11209 (US)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 256 978 B1

Courier Press, Leamington Spa, England.

**Description**

The present invention relates to the use of perfluoroalkyl containing triazoles as oil soluble corrosion inhibitors for metal surfaces and to new perflouroalkyl containing triazoles.

French Patent No. 2 026 493 discloses the reaction products of benzotriazoles and perfluoroalkyl carboxylic acids as oil-soluble corrosion inhibitors.

EP—A—0 088 724 discloses a metal passivator for functional fluids which is a mixture of N-di(hydroxyethyl)aminomethyl-4-methylbenzotriazole and -5-methylbenzotriazole.

More specifically, one embodiment of the present invention relates to a method of inhibiting corrosion on a metal surface by treating said surface with an effective corrosion inhibiting amount of a compound of the formula

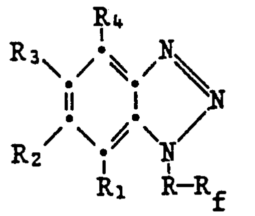

I

or

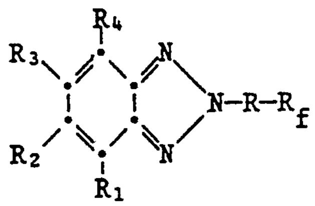

II,

wherein

R is a) an alkylene of up to 16 carbon atoms which is unsubstituted or substituted by hydroxy, alkoxy with up to 7 carbon atoms, or halo, and which may be interrupted by oxygen, sulfur, carboxamido, sulfonamido, aminosulfonyl, aminocarbonyl, carbonyloxy, sulfinyl, or sulfonyl; or

b) R is sulfonyl or carbonylaminoalkylene or -aralkylene of up to 16 carbon atoms, which is unsubstituted or substituted by hydroxy, alkoxy with up to 7 carbon atoms, or halo, and said alkylene is uninterrupted or interrupted by oxygen, sulfur, carboxamido, sulfonamido, aminocarbonyl, aminosulfonyl, carbonyloxy, sulfinyl, or sulfonyl;

$R_f$ is perfluroalkyl of 2 to 16 carbons or perfluoroalkoxyalkyl of 4 to 16 carbon atoms; and $R_1$—$R_4$ are each independently hydrogen, hydroxy or halogen, or are alkyl up to 10 carbon atoms, alkanoyloxy of up to 7 carbon atoms, alkoxy of up to 7 carbon atoms, or alkanoylamino of up to 7 carbon atoms, each of which are unsubstituted or substituted by $C_6$—$C_{10}$-aryl, $C_4$—$C_7$-cycloalkyl, $C_3$—$C_6$-azacycloalkyl, alkoxy of up to 7 carbon atoms, hydroxy, halogen, cyano, or by poly $C_2$- or $C_3$-alkyleneoxy terminated by hydroxy or $C_2$—$C_3$-alkylene and having from 3 to 40 alkylenoxy units, or two or more of adjacent $R_1$—$R_4$ together form fused carbon or heterocyclic rings having 5—8 ring atoms each; or with mixtures thereof.

Preferably, $R_1$, $R_2$, $R_3$ and $R_4$ are hydrogen, alkyl or alkoxy of up to 7 carbon atoms being unsubstituted or substituted as above, preferably unsubstituted. Preferably at least one of $R_1$ to $R_4$ is hydrogen. Most preferably $R_1$—$R_4$ are each hydrogen.

R is preferably unsubstituted or substituted alkylene of 1 to 12, especially of 2 to 10 and in particular 6 to 10 carbon atoms. R may be unsubstituted or preferably substituted by hydroxy and is preferably interrupted independently by one or two oxygen atoms, a sulfur atom, a carboxamido group or a sulfonamido group. Preferably, R is $C_2$—$C_{16}$-alkylene interrupted independently by one or two oxygen atoms or sulfur and is substituted by hydroxy. Most preferably, R is alkylene of 6 to 10 carbon atoms substituted by hydroxy and is interrupted independently by oxygen or sulfur. The substituent on R is preferably hydroxy. In a preferred embodiment R is a $C_2$—$C_{16}$-alkylene interrupted by 1 or 2 oxygen atoms, or a group selected from sulfur, carboxamide and sulfonamide and is substituted by hydroxy.

$R_f$ is preferably perfluoroalkyl of 4 to 16 carbon atoms, most preferably of 6 to 12 carbon atoms. $R_f$ may also represent a mixture of said perfluoroalkyl.

As used throughout this specification, alkyl, alkoxy, alkanoyloxy and alkanoylamino preferably contain up to 4 carbon atoms.

Poly $C_2$—$C_3$-alkyleneoxy, terminated by hydroxy or $C_2$—$C_3$-alkylene preferably contains 3 to 20 alkylenoxy units.

Aryl is preferably phenyl or naphthyl.

Cycloalkyl is preferably cyclopentyl or cyclohexyl.

Azacycloalkyl is preferably pyrrolidinyl such as the 2-, or 3-pyrrolidinyl, or piperidinyl, such as the 2-, 3-,

or 4-piperidinyl wherein the nitrogen thereof is unsubstituted or substituted by alkyl of up to 7 carbon atoms, especially methyl or ethyl.

Where two adjacent $R_1$, $R_2$, $R_3$ or $R_4$ together form fused rings having 5 to 8 ring atoms, the ring atoms preferably consist of carbon and/or nitrogen. Suitable fused rings, taken together with the respective carbon atoms to which $R_1$, $R_2$, $R_3$ and $R_4$ are attached include, for example, the fused benzene, quinoline, benzopyrazine, thiadiazole, thiazole, naphthalene, or bicyclopentadiene.

Also, three adjacent moieities of $R_1$, $R_2$, $R_3$ or $R_4$ can, together with the benzene ring to which they are attached, form a fused ring system, such as acenaphthylene.

Highly preferred are those compounds wherein $R_1$, $R_2$, $R_3$ and $R_4$ independently represent hydrogen, alkyl or alkoxy of up to 7 carbon atoms and, most preferred are those compounds wherein $R_1$, $R_2$, $R_3$ and $R_4$ are hydrogen.

A further embodiment of this invention are compounds of formulae Ia or IIa

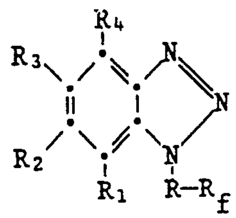

Ia

or

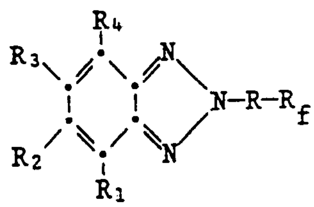

IIa,

wherein

R is alkylene of up to 16 carbon atoms which is unsubstituted or substituted by hydroxy, alkoxy of up to 7 carbon atoms or halo and which may be interrupted independently by one or two oxygen, sulfur, carboxamido, sulfonamido, aminocarbonyl, aminosulfonyl, carbonyloxy, sulfinyl or sulfonyl;

$R_f$ is perfluoroalkyl of 2 to 16 carbon atoms or perfluoroalkoxyalkyl of 4 to 16 carbon atoms; and

$R_1$—$R_4$ are each independently hydrogen, hydroxy or halogen, or are alkyl of up to 10 carbon atoms, alkanoyloxy of up to 7 carbon atoms, alkoxy of up to 7 carbon atoms, or alkanoylamino of up to 7 carbon atoms, each of which are unsubstituted or substituted by $C_6$—$C_{10}$-aryl, $C_4$—$C_7$-cycloalkyl, $C_3$—$C_6$-azacycloalkyl, alkoxy of up to 7 carbon atoms, hydroxy, halogen, cyano, or by poly $_2$- or $C_3$-alkyleneoxy terminated by hydroxy or $C_2$—$C_3$-alkylene and having from 3 to 40 alkylenoxy units, or two or more of adjacent $R_1$—$R_4$ together form fused carbon or heterocyclic rings having 5—8 ring atoms each; or mixtures thereof.

There preferred embodiments mentioned above read also on these compounds.

Compounds of formulae I, Ia, II or IIa can be obtained by reacting triazoles of the formula

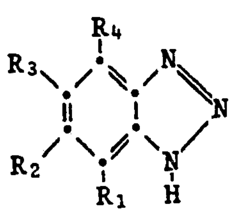

III

with epoxides of the formula

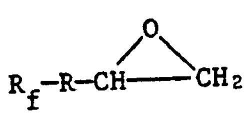

IV.

The reaction is optionally carried out in the presence of a suitable diluent or solvent such as benzene, methylene chloride, chloroform, acetone, diethyl ether, toluene and the like. $R_f$, R, $R_1$, $R_2$, $R_3$, and $R_4$ are as previously defined. If desired, the reaction can be optionally conducted in the presence of a Lewis acid, such as $AlCl_3$ or $BF_3$, at a temperature between preferably about 0°C to about 120°C.

Typical known epoxides which are included within the context of this invention are:

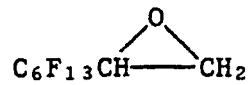

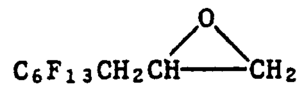

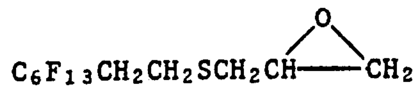

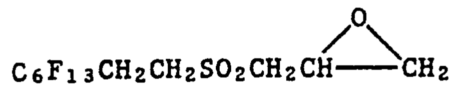

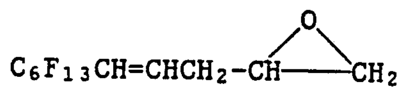

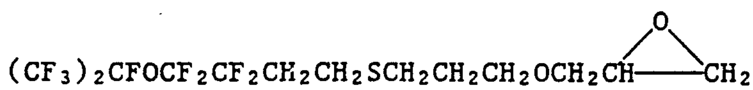

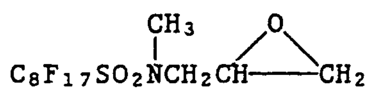

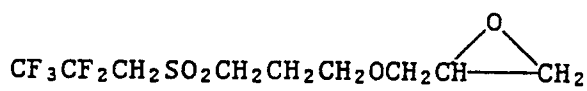

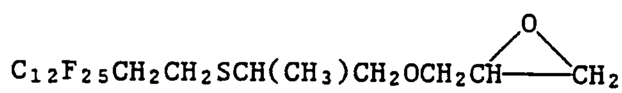

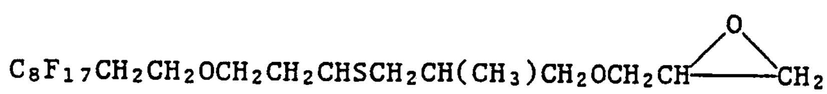

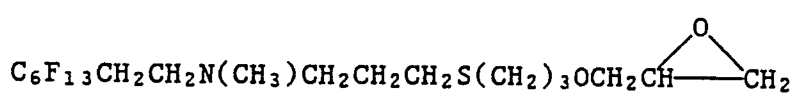

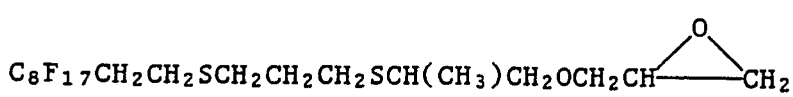

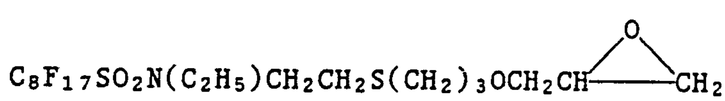

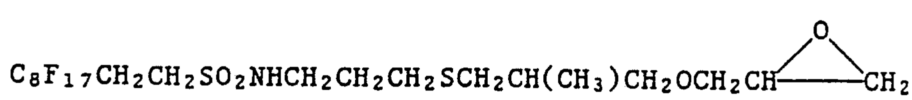 .

Typical known triazoles included within the context of the invention include:

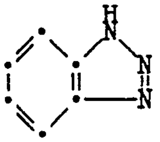 , 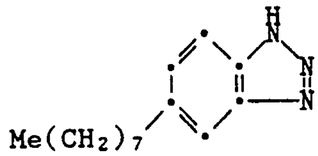 , 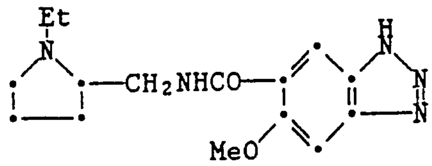 ,

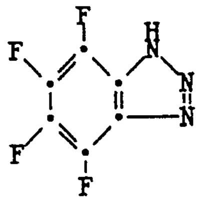 , 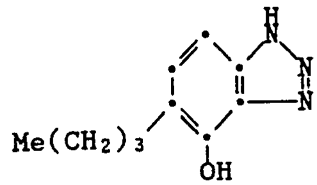 , 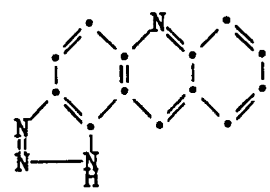 ,

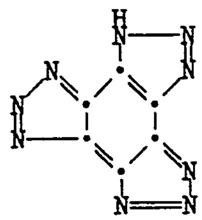 , 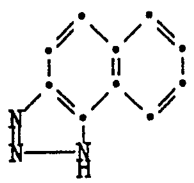 , 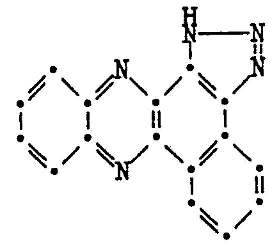 ,

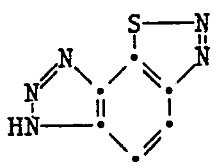 , 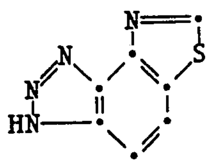 , 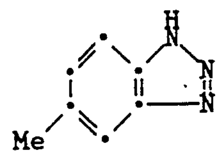 ,

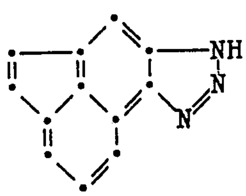 , 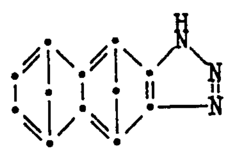 , 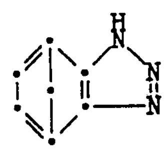 .

Alternatively, the compounds of the instant invention may be prepared by reacting a triazole of formula III with a compound of the formula

$$R_f—R—X \qquad IV$$

where X is a leaving group, and $R_f$ and R are as defined as above.

Suitable leaving groups, X, include for example halo, especially chloro, bromo and iodo. The reaction may be generally conducted at a temperature between about 0°C and 120°C, with removal of the by-product HX, for example by passing an inert gas through the reaction mixture, such as air or nitrogen, or by conducting reaction in the presence of a base, such as an alkali, or alkaline earth metal, -hydroxide, -carbonate or -bicarbonate, or in the presence of ammonia, ammonium hydroxide or an amine, such as triethyl amine.

Suitable compounds of the formula IV include, for example

$C_6F_{13}CH_2CH_2I$, $C_8F_{17}CH_2CH_2I$, $C_6F_{13}CH_2CH_2Br$, $C_6F_{13}CH_2CH_2Cl$,
$C_8F_{17}CH_2CH_2Br$, $C_8F_{17}CH_2CH_2OCH_2CH_2Br$, $C_8F_{17}CH_2CH_2SO_2NHCH_2CH_2Br$,
$C_8C_{17}CH_2CH_2CONHCH_2CH_2Br$, $C_8F_{17}CH_2CH_2SO_2CH_2CH_2Br$, $C_9F_{19}SO_2Cl$,
$C_8F_{17}SO_2Br$, $C_7F_{15}SO_2F$, $C_7F_{15}COCl$, $C_6F_{13}CH_2CH_2SO_2Cl$, $C_6F_{13}CH_2CH_2COCl$.

The compounds of the invention are particularly useful as oil soluble corrosion inhibitors for metals against corrosion from corrosive acids, brines and oxidation. They are suitably employed in compositions having an oil component which will come into contact with at least one of the metals below. Preferably, the oil is selected from lubricating oils, hydrocarbon diluents, such as toluene, xylene, fuel oil and acetone. The preferred concentration of the compounds of formulae I and/or II in the composition is about 0.01% to about 5%, preferably about 0.01 to about 0.1% by weight.

A further embodiment of this invention is a composition containing an oil component and an effective corrosion inhibiting amount of a compound of formulae I and/or II.

Metals for which corrosion is inhibited by the invention include for example iron, steel, copper, brass and preferably are steel or iron.

Their effectiveness of the compounds of formulae I and II as corrosion inhibitors is determined by the following procedure:

Corrosion test coupons [SAE 1010, mild steel, cold-rolled, polished (#280 grit)] are immersed at room temperature in a 0.4% (w/w) toluene solution of the inhibitor sample for 30 minutes. The coupons are then taken out of the solution slowly and dried in ambient air for 30 minutes before weighing. Each weighed coupon is completely immersed in the test solution (5% HCl solution, 110 ml total volume) contained in a glass jar and held at room temperature for 4 hours. The weight loss after the 4 hour period is measured. Triplicate runs are made for each inhibitor sample.

For a clearer understanding of the invention, the following specific examples are given. Unless otherwise specified all parts are by weight.

Example 1

A mixture of 3,01 g benzotriazole (0,0252 mole) and 52,04 g toluene is heated at 80°C until a clear liquid is obtained. Then 10,0 g

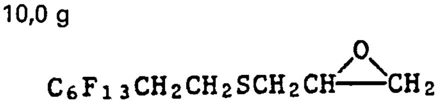 (0,0229 mole)

are added and the reaction mixture is heated at 80°C for 3 hours. Then 0,04 g boron trifluoride etherate are added and the reaction mixture is heated at 80°C for another 26 hours. Removal of the toluene affords a product containing a mixture of two isomers with the following structures I and II, as determined by $^1$H-NMR:

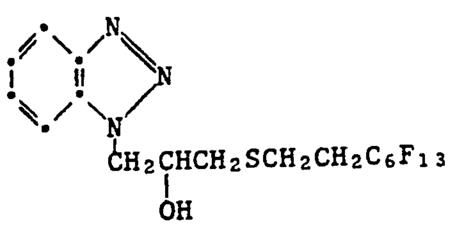 and 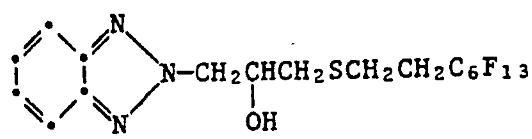

Analysis:
Calculated (percent): C 37,3; H 2,6; N 8,2; F 43,5,
Found (percent) C 37,3; H 2,6; N 8,3; F 42,8.

Example 2

A mixture of 4,37 tolutriazole (0,0326 mole) and 77,48 g toluene is heated at 80°C until a clear liquid is obtained. Then 15,0 g

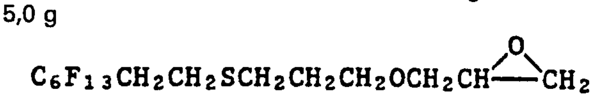 (0,0296 mole)

are added and the reaction mixture is heated at 80°C for 3 hours. Then 0,04 g boron trifluoride etherate are added and the reaction mixture is heated at 80°C for another 26 hours. Removal of the toluene affords a product containing a mixture of isomers with following structures as determined by $^1$H-NMR:

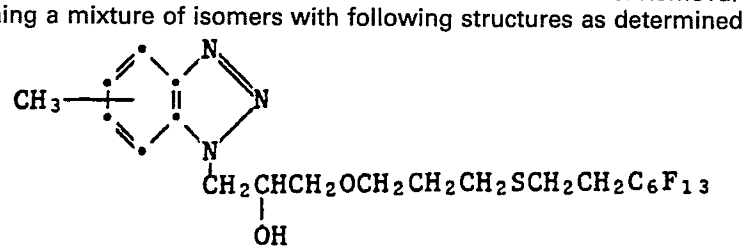 and

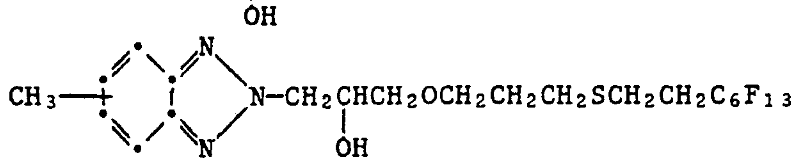

Analysis:

| | | | | |
|---|---|---|---|---|
| Calculated (percent): | C 39,8; | H 3,5; | N 7,1; | F 38,6, |
| Found (percent) | C 41,2; | H 3,6; | N 7,5; | F 37,3. |

Example 3

A mixture of 1,57 g benzotriazole (0,0132 mole) and 24,28 g toluene is heated at 80°C until a clear liquid is obtained. Then 4,5 g of

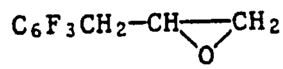

are added and the reaction mixture is heated at 80°C for 2 hours. Then 0.04 g boron trifluoride etherate are added and the reaction mixture is heated at 80°C for another 25 hours. A solid precipitates. It is collected by filtration and washed with hexane. A pale beige product is obtained with the following structure as determined by $^1$H-NMR:

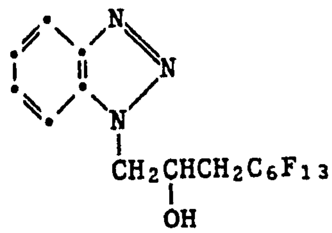

Analysis:

| | | | | |
|---|---|---|---|---|
| Calculated (percent): | C 36,9; | H 2,0; | N 9,1; | F 48,7, |
| Found (percent) | C 36,7; | H 2,0; | N 8,9; | F 49,1. |

Example 4

Compounds from the previous examples are screened as corrosion inhibitors by the method previouslv described.

| Compound from Example | Weight Loss (mg) | % Protection[a] |
|---|---|---|
| 1 | 2.8 ± 0.6 | 93 |
| 2 | 3.4 ± 0.1 | 91 |
| 3 | 24 ± 5 | 38 |

a % protection = $(\Delta W_c - \Delta W_s)\ 100/\Delta W_c$ where $\Delta W_c$ = weight loss of the control coupon, $\Delta W_s$ = weight loss of the coated coupon.

Example 5

A mixture of 0,66 g benzotriazole (0,0054 mole) and 13,40 g chloroform is heated at 60°C until a clear liquid is obtained. The 2,69 g of

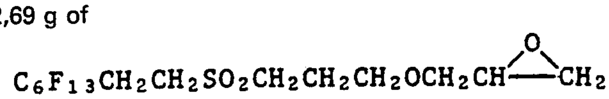

are added and the reaction mixture is heated at 60°C for 2 hours 40 minutes. Removal of the chloroform affords a product with the following structures, as determined by $^1$H-NMR:

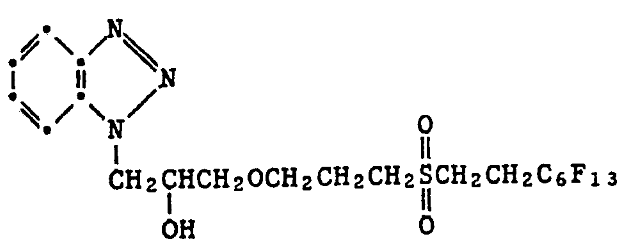

and

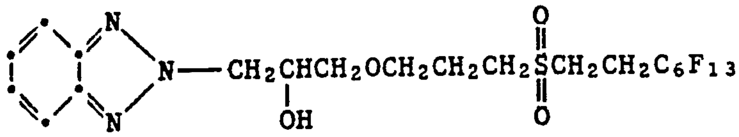

Example 6

A mixture of 2,10 g benzotriazole (0,0176 mole), 2,44 g potassium carbonate (0,0176 mole), and 21,60 g acetone is heated under reflux. A solution of 9,22 g $RfCH_2CH_2I$ (Rf = mixture of $C_2$—$C_8$-perfluoroalkyl) (0,0160 mole) and 33,40 g acetone is added, and the reaction mixture is heated under reflux for 20 hours. A clear liquid is obtained upon filtration. Removal of the acetone affords a solid, which is then added to 0,40 g hexafluoroxylene. The mixture is heated on a steam bath for 30 minutes, filtered to remove insolubles, and then placed under high vacuum (0,065 mbar). The product is a pale brown waxy solid with the following structures as determined by $^1$H-NMR:

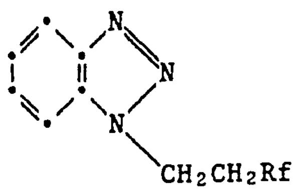

and

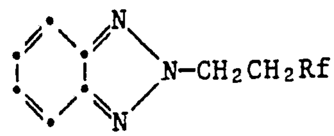

Example 7

Compounds from the previous examples are screened as corrosion inhibitors according to the method previously described.

| Compound from Example | Weight Loss (mg) | % Protection |
|---|---|---|
| 5 | 27 ± 10 | 20 |
| 6 | 24 ± 6 | 28 |

Example 8

A mixture of 1,58 benzotriazole (0,0131 mole) and 7,0 g

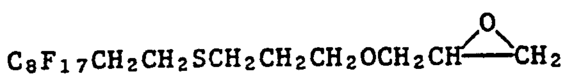 (0,0114 mole)

and 34,24 g toluene, is heated to reflux (110—111°C) for 10 hours. Then 0,02 g boron trifluoride etherate are added and the reaction mixture is heated under reflux for 45 minutes. Removal of the toluene affords a product containing a mixture of two isomers with the following structures as determined by $^1$H-NMR:

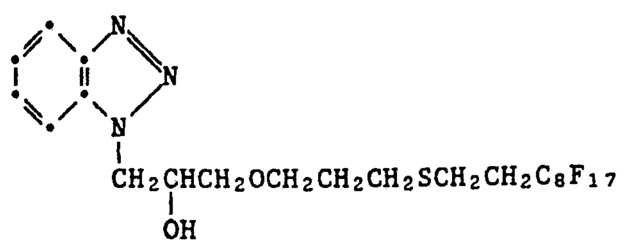

and

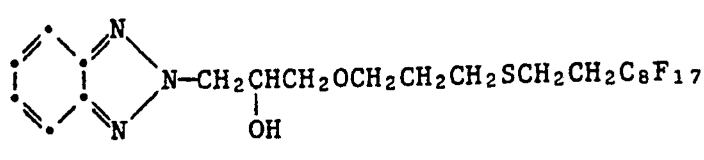

Analysis:

| | | | | |
|---|---|---|---|---|
| Calculated (percent): | C 37,0; | H 2,8; | N 6,7; | F 45,3, |
| Found (percent) | C 37,3; | H 2,8; | N 6,7; | F 43,6. |

Example 9

The compound from example 8 is screened as corrosion inhibitor according to the method previously described.

| Compound from Example | Weight Loss (mg) | % Protection |
|---|---|---|
| 8 | 14 ± 1 | 66 |

Example 10

A mixture of 2,09 g benzotriazole (0,0175 mole) and 22,12 g toluene is heated at 80°C until a clear liquid is obtained. Then 1,94 g triethylene (0,0191 mole) are added followed by the addition of a clear solution 8,81 g perfluorooctanesulfonyl fluoride (0,0173 mole) in 32,0 g hexafluoroxylene. The reaction mixutre is heated at 80°C for 112 hours. Removal of the solvents affords a product with the following structure, as determined by $^1$H-NMR:

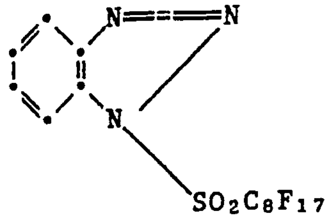

Example 11

5 g toluene diisocyanate (0,0287 mole), 0,05 g triethyl amine (0,0005 mole), and 63,72 g toluene are mixed under nitrogen at room temperature. Over a period of 10 minutes, 10,93 g $C_6F_{13}CH_2CH_2SH$ are added the reaction temperature approaches a maximum of 29°C. The reaction mixture is then stirred at room temperature for 30 minutes. A clear 80°C solution of 3,42 g benzotriazole (0,0287 mole) in 13,68 g toluene is then added over a period of 13 minutes and the reaction temperature reaches a maximum 28°C. The reaction mixture is stirred at room temperature for 45 minutes, at which time the reaction is complete as determined by infrared spectroscopy (absence of N=C=O band at 22—70 cm$^{-1}$). Removal of the solvent affords a white powder product with the following structure as determined by $^{13}$C NMR.

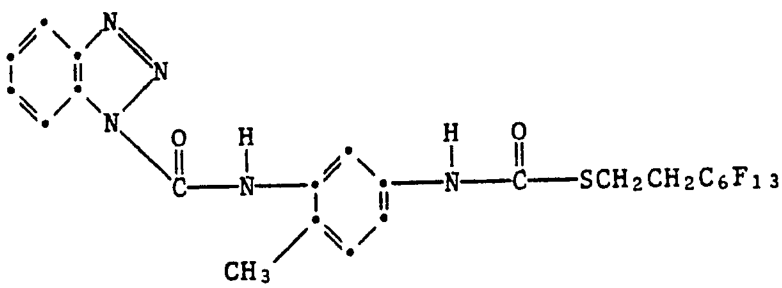

Example 12

Compounds from the previous examples 10 and 11 are screened as corrosion inhibitors according to the method previously described.

| Compound from Example | Weight Loss | % Protection |
|---|---|---|
| 10 | 26 ± 4 | 37 |
| 11 | 13 ± 4 | 61 |

**Claims**

1. A method of inhibiting corrosion on a metal surface comprising treating said surface with an effective amount of a compound of the formula

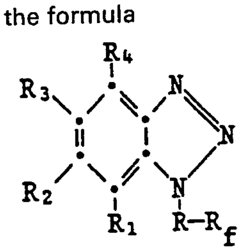

I

or

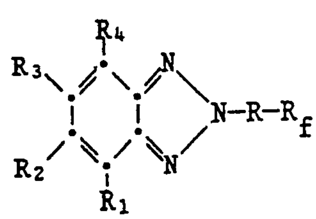

II,

wherein

R is a) an alkylene of up to 16 carbon atoms which is unsubstituted or substituted by hydroxy, alkoxy with up to 7 carbon atoms, or halo, and which may be interrupted by oxygen, sulfur, carboxamido, sulfonamido, aminosulfonyl, aminocarbonyl, carbonyloxy, sulfinyl, or sulfonyl; or

b) R is sulfonyl or carbonylaminoalkylene or -aralkylene of up to 16 carbon atoms, which is unsubstituted or substituted by hydroxy, alkoxy with up to 7 carbon atoms, or halo, and said alkylene is uninterrupted or interrupted by oxygen, sulfur, carboxamido, sulfonamido, aminocarbonyl, aminosulfonyl, carbonyloxy, sulfinyl, or sulfonyl;

$R_f$ is a $C_2$—$C_{16}$-perfluoroalkyl or a $C_4$—$C_{16}$-perfluoroalkoxyalkyl;

$R_1$, $R_2$, $R_3$ and $R_4$ are each independently

a) hydrogen, halogen or hydroxy or b) alkyl of up to 10 carbon atoms, alkoxy of up to 7 carbon atoms, alkanoyloxy of up to 7 carbon atoms or alkanoylamino of up to 7 carbon atoms each of which are independently unsubstituted or further substituted by $C_6$—$C_{10}$-aryl, $C_4$—$C_7$-cycloalkyl, $C_3$—$C_6$-azacycloalkyl, alkoxy of up to 7 carbon atoms, hydroxy, halogen, cyano, or poly $C_2$- or $C_3$-alkyleneoxy terminated by hydroxy or $C_2$—$C_3$-alkylene and having from 3 to 40 alkyleneoxy units or 2 or more of $R_1$—$R_4$ may together form one or more fused rings of 5—8 members each, or with mixtures thereof.

2. The method of claim 1 wherein R is a $C_1$—$C_{12}$ alkylene which is said unsubstituted or substituted.

3. The method of claim 2 wherein R is a $C_2$—$C_{10}$ alkylene which is said unsubstituted or substituted.

4. The method of claim 3 wherein R is a $C_6$—$C_{10}$ alkylene which is said unsubstituted or substituted.

5. The method of claim 1 wherein said R is a $C_2$—$C_{16}$ alkylene interrupted by 1 or 2 oxygen atoms, or a group selected from sulfur, carboxamide and sulfonamide and is substituted by hydroxy.

6. The method of claim 1 wherein said R is a $C_2$—$C_{16}$ alkylene interrupted independently by one or two oxygen atoms or sulfur and is substituted by hydroxy.

7. The method of claim 1 wherein said substituent on said R is hydroxy.

8. The method of claim 1 wherein $R_f$ is $C_4$—$C_{16}$ perfluoroalkyl.

9. The method of claim 1 wherein $R_f$ is $C_6$—$C_{12}$ perfluoroalkyl.

10. The method of claim 1 wherein at least one of $R_1$—$R_4$ is hydrogen.

11. The method of claim 1 wherein $R_1$—$R_4$ are each hydrogen.

12. A compound of the formula

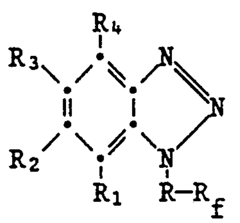

Ia

or

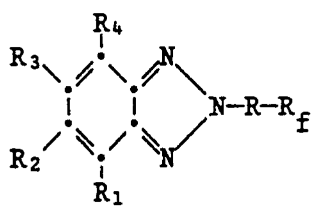

IIa,

R is alkylene of up to 16 carbon atoms which is unsubstituted or substituted by hydroxy, alkoxy with up of to 7 carbon atoms or halo and said alkylene is uninterrupted or interrupted by oxygen, sulfur, carboxamido, sulfonamido, aminocarbonyl, aminosulfonyl, carbonyloxy, sulfinyl and sulfonyl;

$R_f$ is a $C_2$—$C_{16}$-perfluoroalkyl or a $C_4$—$C_{16}$-perfluoroalkoxyalkyl;

$R_1$, $R_2$, $R_3$ and $R_4$ are each independently

a) hydrogen, halogen or hydroxy or b) alkyl of up to 10 carbon atoms, alkoxy of up to 7 carbon atoms, alkanoyloxy of up to 7 carbon atoms or alkanoylamino of up to 7 carbon atoms each of which are independently unsubstituted or further substituted by $C_6$—$C_{10}$-aryl, $C_4$—$C_7$-cycloalkyl, $C_3$—$C_6$-azacycloalkyl, alkoxy of up to 7 crbon atoms, hydroxy, halogen, cyano, or poly $C_2$— or $C_3$— alkyleneoxy terminated by hydroxy or $C_2$—$C_3$-alkylene and having from 3 to 40 alkylenoxy units or 2 or more of adjacent $R_1$—$R_4$ may together form one or more fused carbon or heterocyclic rings of 5—8 members each; or mixtures thereof.

13. A composition comprising an oil component and an effective corrosion inhibiting amount of a compound of formulae I and/or II wherein the groups R to Rf are defined as in claim 1.

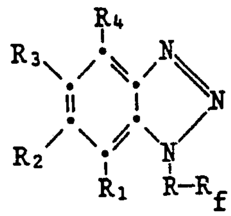

I

or

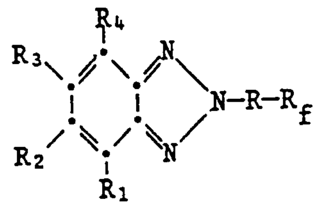

II,

## Patentansprüche

1. Ein Verfahren zur Korrosisonsinhibation auf einer Metalloberfläche, umfassend die Behandlung besagter Oberfläche mit einer wirksamen Menge einer Verbindung der Formeln

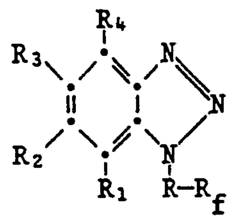

I

oder

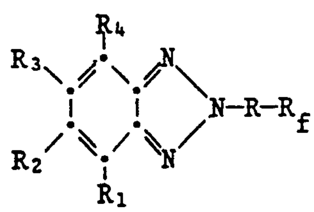

II,

oder deren Mischungen, worin

R a) Alkylen mit bis zu 16 C-Atomen bedeutet, das unsubstituiert oder mit Hydroxy, Alkoxy mit bis zu 7 C-Atomen oder Halogen substituiert ist, und das gegebenenfalls durch Sauerstoff, Schwefel, Carboxamid, Sulfonamid, Aminosulfonyl, Aminocarbonyl, Carbonyloxy, Sulfinyl oder Sulfonyl unterbrochen ist; oder

b) Sulfonyl, Carbonylaminoalkylen oder -aralkylen mit bis zu 16 C-Atomen darstellt, das unsubstituiert oder mit Hydroxy, Alkoxy mit bis zu 7 C-Atomen oder Halogen substituiert ist, und besagtes Alkylen nicht unterbrochen oder mit Sauerstoff, Schwefel, Carboxamid, Sulfonamid, Aminocarbonyl, Aminosulfonyl, Carbonyloxy, Sulfinyl oder Sulfonyl unterbrochen ist;

$R_f$ $C_2$ bis $C_{16}$-Perfluoralkyl oder $C_4$—$C_{16}$-Perfluoralkoxyalkyl bedeutet, und

$R_1$, $R_2$, $R_3$ und $R_4$ je unabhängig voneinander a) Wasserstoff, Halogen oder Hydroxy sind oder b) Alkyl mit bis zu 10 C-Atomen, Alkoxy mit bis zu 7 C-Atomen, Alkanoyloxy mit bis zu 7 C-Atomen oder Alkanoylamino mit bis zu 7 C-Atomen darstellen, die je unabhängig voneinander unsubstituiert oder mit $C_6$—$C_{10}$-Aryl, $C_4$—$C_7$-Cycloalkyl, $C_3$—$C_6$-Azacycloalkyl, Alkoxy mit bis zu 7 C-Atomen, Hydroxy, Halogen, Cyano, oder Polyoxa-$C_2$- oder -$C_3$-Alkylen, das mit Hydroxy oder $C_2$—$C_3$-Alkylen terminiert ist und 3 bis 40 Alkylenoxyeinheiten enthält, substituiert, ist oder 2 oder mehr $R_1$—$R_4$ zusammen einen oder mehr kondensierte Ringe mit je 5—8 Ringgliedern bilden.

2. Verfahren gemäss Anspruch 1, worin R $C_1$—$C_{12}$-Alkylen bedeutet, das unsubstituiert oder substituiert ist.

3. Verfahren gemäss Anspruch 2, worin R $C_2$—$C_{10}$-Alkylen bedeutet, das unsubstituiert oder substituiert ist.

4. Verfahren gemäss Anspruch 3, worin R $C_6$—$C_{10}$-Alkylen bedeutet, das unsubstituiert oder substituiert ist.

5. Verfahren gemäss Anspruch 1, worin R mit 1 oder 2 Sauerstoffatomen, Schwefel, Carboxamid oder Sulfonamid unterbrochenes $C_2$—$C_{16}$-Alkylen ist, das mit Hydroxy substituiert ist.

6. Verfahren gemäss Anspruch 1, worin R $C_2$—$C_{16}$-Alkylen ist, das mit ein oder zwei Sauerstoffatomen oder Schwefel unterbrochen und mit Hydroxy substituiert ist.

7. Verfahren gemäss Anspruch 1, worin R mit Hydroxy substituiert ist.

8. Verfahren gemäss Anspruch 1, worin $R_f$ $C_4$—$C_{16}$-Perfluoralkyl ist.

9. Verfahren gemäss Anspruch 1, worin $R_f$ $C_6$—$C_{12}$-Perfluoralkyl ist.

10. Verfahren gemäss Anspruch 1, worin mindestens eines von $R_1$—$R_4$ Wasserstoff ist.

11. Verfahren gemäss Anspruch 1, worin $R_1$—$R_4$ je Wasserstoff bedeuten.

12. Verbindung der Formeln

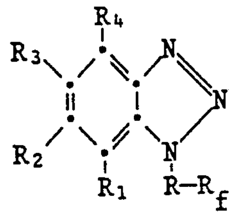

Ia

oder

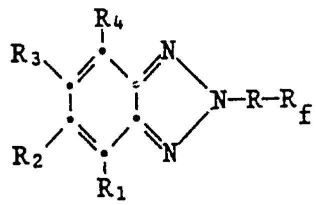

IIa,

oder deren Mischungen, worin

R Alkylen oder Alkenylen mit bis zu 16 C-Atomen bedeutet, das unsubstituiert oder mit Hydroxy, Alkoxy mit bis zu 7 C-Atomen oder Halogen substituiert ist, und das gegebenenfalls durch Sauerstoff, Schwefel, Carboxamid, Sulfonamid, Aminosulfonyl, Aminocarbonyl, Carbonyloxy, Sulfinyl oder Sulfonyl unterbrochen ist;

$R_f$ $C_2$ bis $C_{16}$-Perfluoralkyl oder $C_4$—$C_{16}$-Perfluoralkoxyalkyl bedeuten, und

$R_1$, $R_2$, $R_3$ und $R_4$ je unabhängig voneinander a) Wasserstoff, Halogen oder Hydroxy sind oder b) Alkyl mit bis zu 10 C-Atomen, Alkoxy mit bis zu 7 C-Atomen, Alkanoyloxy mit bis zu 7 C-Atomen oder Alkanoylamino mit bis zu 7 C-Atomen darstellen, die je unabhängig voneinander unsubstituiert oder mit $C_6$—$C_{10}$-Aryl, $C_4$—$C_7$-Cycloalkyl, $C_3$—$C_6$-Azacycloalkyl, Alkoxy mit bis zu 7 C-Atomen, Hydroxy, Halogen, Cyano, oder Polyoxa-$C_2$- oder -$C_3$-Alkylen, das mit Hydroxy oder $C_2$—$C_3$-Alkylen terminiert und 3 bis 40 Alkylenoxyeinheiten enthält, substituiert ist, oder 2 oder mehr benachbarte $R_1$—$R_4$ zusammen einen oder mehr kondensierte Kohlenstoffringe oder heterocyclische Ringe mit je 5—8 Ringgliedern bilden.

13. Zusammensetzung, enthaltend eine Oelkomponente und eine wirksame korrosionsinhibierende Menge einer Verbindung der Formeln I und/oder II

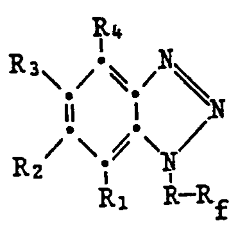

I

oder

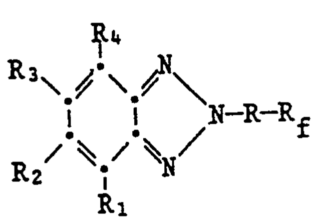

II,

worin die Gruppen R bis $R_f$ wie in Anspruch 1 definiert sind.

**Revendications**

1. Procédé pour inhiber la corrosion sur une surface de métal, comprenant le traitement de ladite surface par une quantité efficace d'un composé de formule

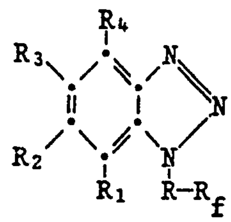

I

ou

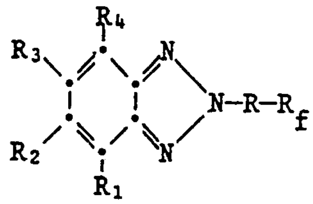

II,

formules dans lesquelles,
R représente

a) un groupe alkylène ayant jusqu'à 16 atomes de carbone, qui n'est pas substitué ou est substitué par un groupe hydroxy, alcoxy ayant jusqu'à 7 atomes de carbone ou halogéno, et qui peut être interrompu par un atome d'oxygène ou de soufre ou par un groupe carboxamido, sulfonamido, aminosulfonyle, aminocarbonyle, carbonyloxy, sulfinyle ou sulfonyle; ou

b) R représente un groupe sulfonyl- ou carbonylaminoalkylène ou -aralkylène ayant jusqu'à 16 atomes de carbone, qui n'est pas substitué ou qui est substitué par un groupe hydroxy, alcoxy ayant jusqu'à 7 atomes de carbone ou halogéno, ledit groupe alkylène étant ininterrompu ou bien étant interrompu par de l'oxygène, du soufre, par un groupe carboxamido, sulfonamido, aminocarbonyle, aminosulfonyle, carbonyloxy, sulfinyle ou sulfonyle;

$R_f$ est un groupe perfluoro-alkyle ayant 2 à 16 atomes de carbone ou un groupe perfluoroalcoxy alkyle ayant 4 à 16 atomes de carbone; et $R_1$ à $R_4$ représentent chacun, indépendamment, un atome d'hydrogène, un groupe hydroxy ou halogéno, ou bien ils représentent chacun un groupe alkyle ayant jusqu'à 10 atomes de carbone, alcanoyloxy ayant jusqu'a 7 atomes de carbone, alcoxy ayant jusqu'à 7 atomes de carbone, ou alcanoylamino ayant jusqu'à 7 atomes de carbone, dont chacun n'est pas substitué ou est substitué par un groupe aryle en $C_6$ à $C_{10}$, cycloalkyle en $C_4$ à $C_7$, azacycloalkyle en $C_3$ à $C_6$, alcoxy ayant jusqu'à 7 atomes de carbone, hydroxy, halogéno, cyano, ou par un groupe polyalkylène oxy(dont le groupe alkylène est en $C_2$ ou $C_3$) terminé par un groupe hydroxy alkylène en $C_2$—$C_3$ et ayant de 3 à 40 motifs alkylène oxy, ou bien deux ou plusieurs des groupes $R_1$ à $R_4$ adjacents forment ensemble des noyaux carbonés ou hétérocycliques condensés ayant chacun 5 à 8 atomes de noyaux, ou avec un de leurs mélanges.

2. Procédé selon la revendication 1, caractérisé en ce que R représente un groupe alkylène en $C_1$ à $C_{12}$, qui est non substitué ou substitué.

3. Procédé selon la revendication 2, dans lequel R représente un groupe alkylène en $C_2$ à $C_{10}$, qui est non substitué ou substitué.

4. Procédé selon la revendication 3, dans lequel représente un groupe alkylène en $C_6$ à $C_{10}$, qui est non substitué ou substitué.

5. Procédé selon la revendication 1, dans lequel ledit symbole R représente un groupe alkylène en $C_2$ à $C_{16}$, interrompu par un ou deux atomes d'oxygène, ou un groupe choisi parmi du soufre, un groupe carboxamide et sulfonamide, et qui est substitué par un groupe hydroxyle.

6. Procédé selon la revendication 1, dans lequel ledit symbole R représente un groupe alkylène en $C_2$ à $C_{16}$ interrompu, indépendamment, par un ou deux atomes d'oxygène ou de soufre et qui est substitué par un groupe hydroxyle.

7. Procédé selon la revendication 1, dans lequel ledit substituant fixé sur le groupe R est un groupe hydroxyle.

8. Procédé selon la revendication 1, dans lequel $R_f$ représente un groupe perfluoroalkyle en $C_4$ à $C_{16}$.

9. Procédé selon la revendication 1, dans lequel $R_f$ représente un groupe perfluoroalkyle en $C_6$ à $C_{12}$.

10. Procédé selon la revendication 1, dans lequel au moins l'un des symboles $R_1$ à $R_4$ représente un atome d'hydrogène.

11. Procédé selon la revendication 1, dans lequel les symboles $R_1$—$R_4$ représentent chacun un atome d'hydrogène.

12. Composé répondant aux formules

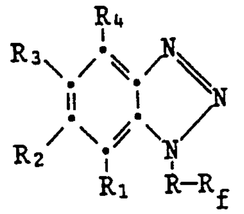

Ia

ou

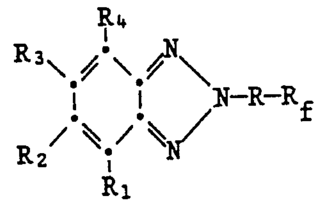

IIa,

dans lesquelles

R représente un groupe alkylène ayant jusqu'à 16 atomes de carbone, qui n'est pas substitué ou est substitué par un groupe hydroxy, alcoxy ayant jusqu'à 7 atomes de carbone ou halogéno, et qui peut être interrompu, indépendamment, par un ou deux atomes d'oxygène ou de soufre, ou groupes carboxamido, sulfonamido, aminocarbonyle, aminosulfonyle, carbonyloxy, sulfinyle ou sulfonyle;

$R_f$ représente un groupe perfluoroalkyle ayant 2 à 16 atomes de carbone ou un groupe perfluoroalkoxyalkyle ayant 4 à 16 atomes de carbone; et

les symboles $R_1$ à $R_4$ représentent chacun, indépendamment, un atome d'hydrogène ou un groupe hydroxy ou halogéno, ou bien un groupe alkyle ayant jusqu'a 10 atomes de carbone, alcanoyloxy ayant jusqu'à 7 atomes de carbone, alcoxy ayant jusqu'à 7 atomes de carbone ou alcanoylamino ayant jusqu'à 7 atomes de carbone, chacun de ces groupes n'étant pas substitué ou étant substitué par un groupe aryle en $C_6$ à $C_{10}$, cycloalkyle en $C_4$ à $C_7$, azacycloalkyle en $C_3$ à $C_6$, alcoxy ayant jusqu'à 7 atomes de carbone, hydroxy, halogéno, cyano, ou par un groupe poly(alkylène oxy), à groupe alkylène en $C_2$ ou $C_3$, terminé par un groupe hydroxy, ou par un groupe alkylène en $C_2$ à $C_3$ comortant de 3 à 40 motifs alkylène oxy, ou bien deux ou plusieurs des groupes $R_1$ à $R_4$ adjacents forment ensemble des noyaux carbonés ou hétérocycliques condensés ayant chacun 5 à 8 atomes de noyau ou leurs mélanges.

13. Composition comprenant un composant huile et une quantité, efficace pour inhiber la corrosion, d'un composé répondant aux formules I et/ou II

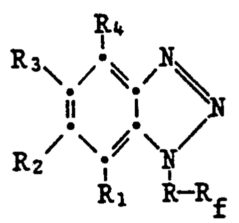

I

ou

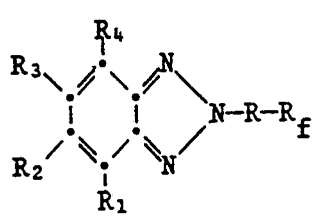

II,

dans lesquelles les groupes $R_1$ à $R_4$ et $R_f$ sont tels que définis à la revendication 1.